# EUROPEAN PATENT SPECIFICATION

(11) **EP 3 213 075 B1**
(45) Date of publication and mention of the grant of the patent: **29.04.2020**
(21) Application number: 15787610.3
(22) Date of filing: 30.10.2015
(51) Int. Cl.: G01N 33/564

(54) **DIAGNOSIS OF AN AUTOIMMUNE DISEASE USING DETECTION OF ANTIBODIES DIRECTED AGAINST C5A-RECEPTOR**
DIAGNOSE EINER AUTOIMMUNERKRANKUNG UNTER VERWENDUNG VON GEGEN C5A-REZEPTOR GERICHTETEN ANTIKÖRPERN
DIAGNOSTIC D'UNE MALADIE AUTO-IMMUNE PAR LA DÉTECTION D'ANTICORPS DIRIGÉS CONTRE LE RÉCEPTEUR C5A

(30) Priority: 31.10.2014 EP 14191208
(43) Date of publication of application: 06.09.2017
(73) Proprietor: CellTrend GmbH, 14943 Luckenwalde (DE)
(72) Inventor: HEIDECKE, Harald, 14169 Berlin (DE); SCHULZE-FORSTER, Kai, 12207 Berlin (DE)
(74) Representative: Vossius & Partner Patentanwälte Rechtsanwälte mbB
(86) International application number: PCT/EP2015/075284
(87) International publication number: WO 2016/066814

(56) References cited:
- US-A1- 2010 144 055
- US-B2- 8 613 926
- ONUMA HIROYUKI ET AL: "Expression of the anaphylatoxin receptor C5aR (CD88) by human articular chondrocytes", RHEUMATOLOGY INTERNATIONAL: CLINICAL AND EXPERIMENTAL INVESTIGATIONS, vol. 22, no. 2, 1 June 2002 (2002-06-01), pages 52-55, XP002569141, SPRINGER, DE ISSN: 0172-8172, DOI: 10.1007/S00296-002-0199-6 [retrieved on 2002-05-08]
- E. GIANNINI ET AL: "Identification of the Major Phosphorylation Sites in Human C5a Anaphylatoxin Receptor in Vivo", JOURNAL OF BIOLOGICAL CHEMISTRY, vol. 270, no. 32, 11 August 1995 (1995-08-11), pages 19166-19172, XP055241402, US ISSN: 0021-9258, DOI: 10.1074/jbc.270.32.19166

### SEQUENCE LISTING

<110> CellTrend GmbH
<120> Diagnosis of an autoimmune disease using detection of antibodies
   directed against C5a-receptor
<130> X3307 PCT BLN
<150> EP 14 19 1208.9
   <151> 2014-10-31
<160> 1
<170> BiSSAP 1.3
<210> 1
   <211> 350
   <212> PRT
   <213> Homo sapiens
<400> 1
   Met Asn Ser Phe Asn Tyr Thr Thr Pro Asp Tyr Gly His Tyr Asp Asp
   1               5                   10                  15
   Lys Asp Thr Leu Asp Leu Asn Thr Pro Val Asp Lys Thr Ser Asn Thr
               20                  25                  30
   Leu Arg Val Pro Asp Ile Leu Ala Leu Val Ile Phe Ala Val Val Phe
           35                  40                  45
   Leu Val Gly Val Leu Gly Asn Ala Leu Val Val Trp Val Thr Ala Phe
       50                  55                  60
   Glu Ala Lys Arg Thr Ile Asn Ala Ile Trp Phe Leu Asn Leu Ala Val
   65                  70                  75                  80
   Ala Asp Phe Leu Ser Cys Leu Ala Leu Pro Ile Leu Phe Thr Ser Ile
                   85                  90                  95
   Val Gln His His His Trp Pro Phe Gly Gly Ala Ala Cys Ser Ile Leu
               100                 105                 110
   Pro Ser Leu Ile Leu Leu Asn Met Tyr Ala Ser Ile Leu Leu Leu Ala
           115                 120                 125
   Thr Ile Ser Ala Asp Arg Phe Leu Leu Val Phe Lys Pro Ile Trp Cys
       130                 135                 140
   Gln Asn Phe Arg Gly Ala Gly Leu Ala Trp Ile Ala Cys Ala Val Ala
   145                 150                 155                 160
   Trp Gly Leu Ala Leu Leu Leu Thr Ile Pro Ser Phe Leu Tyr Arg Val
                   165                 170                 175
   Val Arg Glu Glu Tyr Phe Pro Pro Lys Val Leu Cys Gly Val Asp Tyr
               180                 185                 190
   Ser His Asp Lys Arg Arg Glu Arg Ala Val Ala Ile Val Arg Leu Val
           195                 200                 205
   Leu Gly Phe Leu Trp Pro Leu Leu Thr Leu Thr Ile Cys Tyr Thr Phe
       210                 215                 220
   Ile Leu Leu Arg Thr Trp Ser Arg Arg Ala Thr Arg Ser Thr Lys Thr
   225                 230                 235                 240
   Leu Lys Val Val Val Ala Val Val Ala Ser Phe Phe Ile Phe Trp Leu
                   245                 250                 255
   Pro Tyr Gln Val Thr Gly Ile Met Met Ser Phe Leu Glu Pro Ser Ser
               260                 265                 270
   Pro Thr Phe Leu Leu Leu Asn Lys Leu Asp Ser Leu Cys Val Ser Phe
           275                 280                 285
   Ala Tyr Ile Asn Cys Cys Ile Asn Pro Ile Ile Tyr Val Val Ala Gly
       290                 295                 300
   Gln Gly Phe Gln Gly Arg Leu Arg Lys Ser Leu Pro Ser Leu Leu Arg
   305                 310                 315                 320
   Asn Val Leu Thr Glu Glu Ser Val Val Arg Glu Ser Lys Ser Phe Thr
                   325                 330                 335
   Arg Ser Thr Val Asp Thr Met Ala Gln Lys Thr Gln Ala Val
               340                 345                 350

## Description

### Field

The present disclosure relates to the field of medicine, in particular to the field of diagnostics and prognosis of autoimmune diseases. Furthermore, it relates to methods, means and kits for diagnosis of autoimmune diseases and for the detection of C5a-receptor antibodies in samples of patients.

### Background

Autoimmune diseases arise from an abnormal immune response of the body against substances and tissues normally present in the body (autoimmunity). This may be restricted to certain organs, involve a particular tissue in different places or may even be systemic. The treatment of autoimmune diseases typically involves immunosuppression-medication that decreases the immune response. A large number of autoimmune diseases are recognized. A major understanding of the underlying pathophysiology of autoimmune diseases has been the application of genome wide association scans that have identified a striking degree of genetic sharing among the autoimmune diseases (Cotsapas C, Hafler DA (2013). "Immune-mediated disease genetics: the shared basis of pathogenesis". Trends in Immunology 34 (1): 22-6).

It has been estimated that autoimmune diseases are among the ten leading causes of death among women in all age groups up to 65 years (Noel R. Rose and Ian R. MacKay, "The Autoimmune Diseases" fourth edition). A substantial minority of the population suffers from these diseases, which are often chronic, debilitating, and life-threatening.

Collagenosis or connective tissue autoimmune disease is the term for a group of rare autoimmune diseases. Here the body creates antibodies against parts of the connective tissue. Connective tissue autoimmune diseases are diseases such as SLE, systemic lupus, Wegener granulomatosis, CREST-Syndrome and SHARP-Syndrome. Like other autoimmune diseases, connective tissue autoimmune diseases are difficult to diagnose.

Approximately 100 million people in Europe suffer from some form of inflammatory or degenerative rheumatic disease causing the impact of rheumatic diseases on European societies to be overwhelming for society. Joint diseases account for half of all chronic conditions in persons aged 65 and over. The quality of life of approximately 7,5% of the European population is severely and permanently reduced by pain and functional impairment caused by inflammatory and rheumatic diseases. Immobility and reduced life expectancy are the most drastic consequences of these at present, incurable diseases. In Europe alone, rheumatic diseases impose an economic burden of more than 200 billion EUR per year. Indeed, the impact of rheumatic diseases in a social and economic burden will increase dramatically as the European population ages. A new therapy is targeting the molecules involved in the pathogenesis of chronic inflammatory disease have been developed in recent years. Despite these efforts we are still not able to cure the majority of rheumatic diseases. A therapeutic challenge includes chronicity of inflammation, autoimmunity, and degenerating muscular skeletal system. Although, rheumatic diseases differ in their immunopathology, they share common mechanisms of initiation and perpetuation. Moreover, there is a considerable translational potential for the understanding of other diseases involving the immune system, e.g. autoimmune diseases, allergy and infection. The diversity of rheumatic diseases and the multidisciplinary approach require to understand the molecule basis of these diseases make their diagnosis very difficult.

Symptomatic diagnosis of arthritis and other rheumatic diseases is often difficult, as many symptoms are similar among the different diseases. To make an accurate diagnosis, a physician may need to conduct a review of the medical history, perform a physical examination obtain laboratory tests, x-rays and other imaging tests. The rheumatic diseases are, e.g. rheumatoid arthritis, fibromyalgia, lupus erythematodes, polymyalgia rheumatica, progressive systemic sclerosis, Sjögren's syndrome, systemic lupus erythematosus and joint inflammation.

The American College of Rheumatology has defined (1987) a number of criteria for the diagnosis of, e.g. rheumatoid arthritis. Morning stiffness of more than one hour, arthritis and soft tissue swelling of more than 3 of 14 joints/joint groups, arthritis of hand joints, symmetric arthritis, subcutaneous nodules in specific blazes, rheumatoid factor at a level above the 95 th/percentile and radiological changes suggested of joint erosion. At least four criteria have to be met to establish the diagnosis, although many patients are treated despite not meeting the criteria. When rheumatoid arthritis is being clinically suspected immunological studies are required such as rheumatoid factor (RF, a specific antibody). A negative RF does not rule out rheumatoid arthritis. Recently, a new serological test has been developed, which tests for the presence of so called anti-citrullinated protein (ACP) antibodies. Like RF, this test can not detect all RA patients, and is rarely also positive in non-RA patients. Hence, several other blood tests are usually done to allow for other causes of arthritis, such as systemic lupus erythematosus. Such tests include the erythrocyte sedimentation rate (ESR), cis-reactive protein, full-blood count, renal function, liver enzymes and immunological tests, e.g. antinuclear antibody/ANA are all performed at this stage.

As outlined for rheumatoid arthritis as one disease amongst those in the family, diagnosis in the field of autoimmune diseases is difficult. Thus, there is a need for a safe and reliable diagnostic tool allowing for an improvement in diagnosis, e.g. through combination with other diagnostic tools.

Glomerulonephritis, also known as glomerular nephritis, is a term used to refer to renal diseases affecting both kidneys. The diseases are characterized by inflammation either of the glomeruli or small blood vessels in the kidneys.

Glomerulonephritis' presentation depends on the specific disease entity. It may present with isolated hematuria and/or proteinuria (blood or protein in the urine) or as a nephrotic syndrome, a nephritic syndrome, acute renal failure, or chronic renal failure.

They are categorized into several different pathological patterns. Diagnosing the pattern of GN is important because the outcome and treatment differs in different types. Primary causes are intrinsic to the kidney. Secondary causes are associated with systemic disorders (SLE, vasculitis).

Glomerulonephritis refers to an inflammation of the glomerulus, which is the unit involved in filtration in the kidney. This inflammation typically results in one or both of the nephrotic or nephritic syndromes (Davidson's principles and practice of medicine. (21st ed.). Edinburgh: Churchill Livingstone/Elsevier. 2010. ISBN 978-0-7020-3084-0).

The nephrotic syndrome is characterized by the finding of edema in a patient that has increased protein in the urine and decreased protein in the blood, with increased fat in the blood. Inflammation that affects the cells surrounding the glomerulus, podocytes, increases the permeability to proteins, resulting in an increase in excreted proteins. When the amount of proteins excreted in the urine exceeds the liver's ability to compensate, fewer proteins are detected in the blood-in particular albumin, which makes up the majority of circulating proteins. With decreased proteins in the blood, there is a decrease in the oncotic pressure of the blood. This results in edema, as the oncotic pressure in tissue remains the same. This is worsened by the secretion of the hormone Aldosterone by the adrenal gland, which is secreted in response to the decrease in circulating blood and causes sodium and water retention. Hyperlipidemia is thought to be a result of the increased activity of the liver (and others (2007). Robbins basic pathology (8th ed.). Philadelphia: Saunders/Elsevier. ISBN 978-1-4160-2973-1).

Podocytes, cells which line the glomerulus, are negatively charged and have small gaps, preventing the filtration of large molecules. When damaged by inflammation, this can result in an increased permeability to proteins.

The nephritic syndrome is characterized by blood in the urine and a decrease in the amount of urine in the presence of hypertension. In this syndrome, inflammatory damage to cells lining the glomerulus are thought to result in destruction of the epithelial barrier, leading to blood being found in the urine. At the same time, reactive changes may result in a decrease in renal perfusion, resulting in a decrease in the production of urine. The renin-angiotensin system may be subsequently activated, because of the decrease in perfusion of juxtaglomerular apparatus, which may result in hypertension.

Membranous glomerulonephritis may cause either nephrotic or a nephritic picture and it is often associated with auto-antibodies to phospholipase A2 receptor.

Rapidly progressive glomerulonephritis, also known as *Crescentic GN,* is characterized by a progressive, rapid deterioration in kidney function. Patients with rapidly progressive glomerulonephritis may present with a nephritic syndrome. In management, steroid therapy is sometimes used, although the prognosis remains poor (COUSER, W (1 May 1999). "Glomerulonephritis". The Lancet 353 (9163): 1509-1515). Three main subtypes are recognized.

Type 1 is Goodpasture syndrome, an autoimmune disease also affecting the lung. In Goodpasture syndrome, IgG-antibodies directed against the glomerular basement membrane trigger an inflammatory reaction, causing a nephritic syndrome and the coughing up of blood. High dose immunosuppression is required (intravenous methylprednisolone) and cyclophosphamide, plus plasmapheresis. Immunohistochemistry staining of tissue specimens shows linear IgG deposits.

Type 2 is characterized by immune-complex-mediated damage, and may be associated with systemic lupus erythematosus, post-infective glomerulonephritis, IgA nephropathy, and Henoch-Scholein purpura.

Type 3 rapidly progressive glomerulonephritis, also called *pauciimmune type,* is associated with causes of vascular inflammation including Wegener granulomatosis and microscopic polyangitis. No immune deposits can be seen on staining, however blood tests may be positive for the ANCA antibody.

Some forms of glomerulonephritis are diagnosed clinically, based on findings on history and examination. Other tests may include urine examination, blood tests investigating the cause, including FBC, inflammatory markers and special tests including ASLO, ANCA, Anti-GBM, Complement levels, antinuclear antibodies, biopsy of the kidney.

However, there is a need for a reliable diagnostic tool for diagnosis of glomerulonephritis allowing for an improvement in diagnosis, e.g., through combination with other diagnostic tools.

Onuma et al. (Rheumatol Int, (2002) 22:52-55) relates to the expression of anaphylatoxin receptor (C5aR (CD88) on chondrocytes of cartilage from patients with rheumatoid arthritis, osteoarthritis, and bone fracture. US 8,613,926 B2 relates to the screening of human anti-human C5aR containing supernatants with C5aR and APC-conjugated F(ab')2 goat anti-human IgG. US 2010/144055 A1 relates to anti-C5aR antibodies and their use for immunological disorders. Giannini et al. (The Journal of Biological Chemistry (1995) 270(32):19166-19172) relates to the identification of the major phosphorylation sites in human C5a anaphylatoxin receptor *in vivo.*

### Summary

The inventors now for the first time identified auto-antibodies directed against the C5a-receptor. Furthermore, it has been observed that the presence of such antibodies is indicative of the presence of an autoimmune disease. The data presented herein demonstrate that auto-antibodies directed against C5a-receptor provide an advantageous tool for diagnosis of autoimmune diseases.

Hence, the present disclosure relates to a method for diagnosis of an autoimmune disease, comprising the step of determining the presence or absence of antibodies directed against C5a-receptor in a sample of the subject to be diagnosed, wherein the presence of antibodies directed against C5a-receptor is indicative of an autoimmune disease in said subject.

In some instances levels may be determined and compared to control levels, as further outlined herein below. Hence, the disclosure further pertains to a method for diagnosis of an autoimmune disease comprising determining the level of antibodies directed against C5a-receptor in a sample of a subject to be diagnosed, wherein a level of antibodies directed against C5a-receptor in the sample of the subject to be diagnosed higher than a control level is indicative of an autoimmune disease, preferably a level of higher/more than 1.1 fold, 1.2 fold, 1.3 fold, 1.4 fold and most preferred 1.5 fold as compared to a control level derived from one or more subjects not having an autoimmune disease is indicative of an autoimmune disease in the subject to be diagnosed.

The invention relates to the embodiments defined in the claims.

In particular, the present invention relates to a method for in vitro diagnosis of an autoimmune disease, comprising the steps of (i) determining the level of autoantibodies directed against Complement component 5a receptor 1 (C5a receptor) in a bodily fluid sample obtained from a subject to be diagnosed, and (ii) comparing the determined level in the bodily fluid sample to a control level of C5a receptor autoantibodies derived from one or more subjects without an autoimmune disease; wherein an increased level in the bodily fluid sample from the subject to be diagnosed as compared to the control level is attributed to the presence of autoantibodies directed against C5a receptor, and is indicative of an autoimmune disease in the subject to be diagnosed.

The levels of antibodies directed against C5a-receptor may also be compared to previously fixed values, e.g. standardized units. One approach to fix such units is outlined herein in greater detail. According to one embodiment, the invention also pertains a method for in vitro diagnosis of an autoimmune disease, wherein the level of antibodies directed against C5a-receptor is determined in a sample from a subject to be diagnosed and wherein a level of anti-C5a-receptor antibodies above 9 units/ml is indicative of an autoimmune disease, preferably above 10 units/ml, more preferably above 12, further preferred above 15 units/ml.

The inventors for the first time show the presence of (auto)antibodies directed against C5a-receptor in samples of subjects. The antibodies in said samples may be detected through there ability to bind C5a-receptor or an antigenic fragment thereof. Hence, the present not claimed disclosure also relates to a method for detecting an anti-C5a-receptor antibody in a sample from a subject, comprising the steps of (a) contacting the bodily fluid sample suspected of comprising an anti-C5a-receptor antibody with C5a-receptor or an antigenic peptide fragment thereof under conditions allowing for the formation of a complex between the antiC5a-receptor antibody with C5a-receptor or the antigenic peptide fragment thereof, and (b) detecting the complex. The assay is preferably an immunoassay.

In the context of the present not claimed disclosure the C5a-receptor or an antigenic peptide fragment thereof can thus be used for the in vitro diagnosis of an autoimmune disease.

The present disclosure further relates to research and/or diagnostic kit for the diagnosis of an autoimmune disease, wherein the kit comprises C5a-receptor or an antigenic (immunogenic) peptide fragment thereof.

### Description of the Drawings

**Fig. 1****:** Standard curve of C5a-receptor autoantibody ELISA. Details see Example 1.
**Fig. 2****:** (A) Comparison of the mean level of anti-C5a-receptor antibodies (units/ml) in serum samples of healthy subjects (HD), patients suffering from rheumatoid arthritis (RA), patients suffering from systemic lupus erythematosus, patients suffering from systemic sclerosis (SKL) and patients suffering from Sjögren's syndrome (SS). Exact values and P-values are given in Table 1. Bars indicate standard error of mean.

### Detailed Description

The present invention is based on the surprising finding that in samples of patients with an autoimmune disease (auto)antibodies directed against C5a-receptor can be detected. In other words the inventors have found that patients with an autoimmune disease have a higher level of antibodies directed against C5a-receptor in the blood (e.g. determined in serum) as control groups not having an autoimmune disease.

The present invention is based on the finding of that levels of auto-antibodies directed against C5a-receptor in subjects have diagnostic and predictive properties. The antibodies to be detected in connection with the present disclosure are therefore autoantibodies, i.e. those produced by immune system of the subject to be diagnosed or to be treated.

Determination of the presence of said antibodies may also be conducted via determining the level of antibodies directed against C5a-receptor. If the level of antibodies is above a certain threshold, presence of antibody is given. Such threshold may be dependent on the actual assay used. In a preferred aspect presence is attributed to a level which is significantly higher than the background (noise) of the used assay. In a further aspect of the diagnostic method, the presence is determined through the comparison of the level of antibodies directed against C5a-receptor in the sample of the subject to be diagnosed to a control level. Such control level may for example be the level obtained in samples of subjects not having an autoimmune disease. In one aspect of the present disclosure determining the presence or absence of antibodies directed against C5a-receptor comprises the steps of (i) determining the level of antibodies directed against C5a-receptor in a sample from a subject to be diagnosed, and (ii) comparing the determined level in the sample to a control level of antibodies directed against C5a-receptor derived from subjects without an autoimmune disease; wherein an increased level in the sample from the subject to be diagnosed as compared to the control level is attributed to the presence of antibodies directed against C5a-receptor in the subject to be diagnosed, and wherein a level equal or decreased level in the sample from the subject to be diagnosed as compared to the control level is attributed to the absence of antibodies directed against C5a-receptor.

The disclosure also relates to a method for the diagnosis of an autoimmune disease, comprising the steps of (i) determining the level of antibodies directed against C5a-receptor in a sample from a subject to be diagnosed; and (ii) comparing the determined level in the sample to a control level of antibodies directed against C5a-receptor derived from subjects without an autoimmune disease; optionally from one or more samples of said subjects without an autoimmune disease; wherein an increased level in the sample from the subject to be diagnosed as compared to the control level is indicative of an autoimmune disease in the subject. It will be understood by those of ordinary skills in the art, that if a preferred autoimmune disease is chosen to be diagnosed, the control level should be derived from subjects without an autoimmune disease, preferably the specific autoimmune disease to be diagnosed, i.e. if systemic lupus erythematosus is to be diagnosed, the control level shall be derived from subjects without systemic lupus erythematosus. Nevertheless, the control level is preferably derived from subjects without any autoimmune disease.

The skilled person will also understand that the "control" level may be implicated in the used assay for detecting said autoantibodies. The skilled person hence may chose particulars of the assay so that the test is positive for the presence of the antibody in the sample if levels above a certain level is reached and *vice versa* be negative for the presence of said autoantibody if levels are determined that are below the control value. In one aspect the present disclosure relates to a method for diagnosis of autoimmune disease in a subject, comprising the steps of determining the absence or presence of antibodies directed against C5a-receptor in a sample from a subject to be diagnosed, wherein the presence of antibodies directed against C5a-receptor in the sample from the subject to be diagnosed are indicative of the presence of the autoimmune disease in said patient.

The terms "anti-C5a-receptor antibody", "C5a-receptor antibody", "antibody directed against C5a-receptor" and "C5a-receptor auto-antibody" are used synonymously herein and refer to the antibody that are present in subjects and that specifically bind to the C5a-receptor or an immunogenic (antigenic) fragment thereof; preferably the anti-C5a-receptor antibody refers to antibodies present in samples from patients having an autoimmune disease as defined herein and specifically binding to the C5a-receptor or an immunogenic fragment thereof. In this context the term "specific binding" refers to antibodies raised against peptides derived from C5a-receptor. Such peptides can comprise additional or less N- or C-terminal amino acids. An antibody is considered to be specific to C5a-receptor or an immunogenic peptide thereof, if its affinity towards the variant it is at least 50-fold higher, preferably 100-fold higher, more preferably at least 1000-fold higher than towards other proteins or peptides. It is well known in the art how to determine binding of antibodies to a specific antigen. Autoantibodies directed against C5a-receptor (anti-C5a-receptor antibodies) are not known until today. The inventors of the present application for the first time demonstrate the presence of such antibodies as well as the diagnostic and predictive value. It was found that the presence and/or increase in the level of antibodies directed against C5a-receptor in samples of a subject to be diagnosed are indicative of the presence of an autoimmune disease.

As described herein, the terms "C5a-receptor" or "C5aR" are interchangeably used herein and refer to the receptor specifically binding the human polypeptide C5a. The C5a receptor also known as complement component 5a receptor 1 (C5AR1), C5a anaphylatoxin chemotactic receptor 1, or CD88 (Cluster of Differentiation 88) is a G protein-coupled receptor for C5a. It functions as a complement receptor for C5a. The skilled artisan will acknowledge that the term "C5a-receptor" as used herein refers to all alleles, preferably to all alleles present in human.

C5a is a protein fragment released from cleavage complement component C5 by protease C5-convertase into C5a and C5b fragments. C5b is important in late events of complement cascade, whereas C5a acts as highly inflammatory peptide. The origin of C5 is in the hepatocyte but its synthesis can also be found in macrophages that may cause local increase of C5a. C5a has chemotactic and anaphylatoxic properties, it is essential in the innate immunity but it is also linked with the adaptive immunity. The increase production of C5a is connected with a number of inflammatory diseases (Manthey HD, Woodruff TM, Taylor SM, Monk PN (2009). "Complement component 5a (C5a).". Int J Biochem Cell Biol 41 (11): 2114-2117). Human polypeptide C5a contains 74 amino acids and has 11kDa. NMR spectroscopy proved that the molecule is composed of four helices and connected by peptide loops with three disulphide bonds between helix IV and II, III. There is a short 1.5 turn helix on N terminus but all agonist activity take place in the C terminus. C5a is rapidly metabolized by a serum enzyme carboxypeptidase B to a 72 amino acid form C5a des-Arg without C terminal arginine.

C5a is an anaphylatoxin and causes increased expression of adhesion molecules on endothelium as well as contraction of smooth muscle and increased vascular permeability. C5a interacts with receptor protein C5aR on the surface of target cells such as macrophages, neutrophils and endothelial cells. C5aR is a member of the G-protein-coupled receptor superfamily of proteins, predicted to have seven trans-membrane helical domains of largely hydrophobic amino acid residues, forming three intra- and three extra-cellular loops, with an extracellular N-terminus and an intracellular C-terminus. C5a-binding to the receptor is a two-stage process: an interaction between basic residues in the helical core of C5a and acidic residues in the extracellular N-terminal domain allows the C-terminus of C5a to bind to residues in the receptor trans-membrane domains. The latter interaction leads to receptor activation, and the transduction of the ligand binding signal across the cell plasma membrane to the cytoplasmic G protein Gi type GNAI2. Sensitivity of C5aR to C5a stimulation is enhanced by Lipopolysaccharides exposure, yet this is not due to C5aR upregulation (Raby AC, Holst B, Davies J, Colmont C, Laumonnier Y, Coles B, Shah S, Hall J, Topley N, Khol J, Morgan BP, Labeta MO (2011). "TLR activation enhances C5a-induced pro-inflammatory responses by negatively modulating the second C5a-receptor, C5L2.". European Journal of Immunology 41 (9): 2741-2752). C5L2 is another C5a-receptor that is thought to regulate the C5a-C5aR effects. There is apparently contradictory evidence showing decoy receptor activity conferring anti-inflammatory properties and also signaling activity conferring pro-inflammatory properties (Manthey HD, Woodruff TM, Taylor SM, Monk PN (2009). "Complement component 5a (C5a)", Int J Biochem Cell Biol 41 (11): 2114-2117; and Klos A, Wende E, Wareham KJ, Monk PN. (2013). "International Union of Pharmacology. LXXXVII. Complement peptide C5a, C4a, and C3a receptors". Pharmacol. Rev. 65 (1): 500-543)

The term "autoimmune disease" as described herein refers to any disease associated with an abnormal immune response of the body against substances and tissues normally present in the body (autoimmunity). This may be restricted to certain organs, involve a particular type of tissue in different places (e.g. connective tissue) or being systemic. The skilled person knows ways to characterize autoimmune diseases as such. He may for example use Witebsky's postulates (first formulated by Ernst Witebsky and colleagues in 1957 and modified in 1994; see Witebsky E, et al. (1957), "Chronic thyroiditis and autoimmunization". J. Am. Med. Assoc. 164 (13): 1439-1447; and Rose NR et al. (September 1993). "Defining criteria for autoimmune diseases (Witebsky's postulates revisited)". Immunol. Today 14 (9): 426-430). These postulates include direct evidence from transfer of pathogenic antibody or pathogenic T cells, indirect evidence based on reproduction of the autoimmune disease in experimental animals, circumstantial evidence from clinical clues, and genetic architecture clustering with other autoimmune diseases. Furthermore, it is possible to classify autoimmune diseases by corresponding type of hypersensitivity: type II, type III, or type IV. While no type of autoimmune disease mimics type I hypersensitivity (see Parham, Peter (2005), The immune system. NY: Garland Science. p. 344, ISBN 0-8153-4093-1). As described herein, "autoimmune disease" refers to an autoimmune disease associated with a particular type of tissue in different places of the body, preferably an autoimmune disease associated with connective tissue. Connective tissue is a kind of animal tissue that supports, connects, or separates different types of tissues and organs of the body. It is one of the four general classes of animal tissues. Connective tissue is found everywhere including in the central nervous system. It is located in between other tissues. All connective tissue has three main components: ground substances, fibers and cells. Preferred connective tissues are tissues comprising collagenous fibers. Due to this collagenous fibers autoimmune disease associated with connective tissue are also summarized as collagenosis. Hence, in one embodiment the autoimmune disease is a collagenosis, preferably selected from the group consisting of systemic lupus erythematosus (SLE), rheumatoid arthritis, systemic sclerosis, Sjögren's syndrome, mixed connective tissue disease, eosinophilia infectiosa, polymyositis, dermatomyositis, periarteriitis nodosa, Wegener-granulomatose, CREST-syndrome and SHARP-syndrome, more preferred selected from the group consisting of systemic lupus erythematosus (SLE), rheumatoid arthritis, systemic sclerosis, and Sjörgen's syndrome.

Hence, in one aspect the disclosure relates to a method for diagnosing collagenosis, comprising the step of determining the presence or absence of antibodies directed against C5a-receptor in a sample of the subject to be diagnosed, wherein the presence of antibodies directed against C5a-receptor is indicative of a collagenosis in said subject. In yet a further aspect the disclosure relates to a method for diagnosing a collagenosis selected from the group consisting of systemic lupus erythematosus (SLE), rheumatoid arthritis, systemic sclerosis, and Sjögren's syndrome, comprising the step of determining the presence or absence of antibodies directed against C5a-receptor in a sample of the subject to be diagnosed, wherein the presence of antibodies directed against C5a-receptor is indicative of a collagenosis in said subject, said collagenosis being selected from the group consisting of systemic lupus erythematosus (SLE), rheumatoid arthritis, systemic sclerosis, and Sjögren's syndrome.

Furthermore, the disclosure relates to a method for diagnosing glomerulonephritis, comprising the step of determining the presence or absence of antibodies directed against C5a-receptor in a sample of the subject to be diagnosed, wherein the presence of antibodies directed against C5a-receptor is indicative of a collagenosis in said subject.

As can be derived from the data enclosed herein, levels of antibodies directed against C5a-receptor are particularly increased in patients suffering from systemic lupus erythematosus (SLE). Therefore, in one preferred aspect the present disclosure relates to a method for diagnosing systemic lupus erythematosus (SLE) comprising the step of determining the presence or absence of antibodies directed against C5a-receptor in a sample of the subject to be diagnosed, wherein the presence of antibodies directed against C5a-receptor is indicative of systemic lupus erythematosus (SLE) in said subject.

However, the disclosure likewise relates to a method for diagnosing rheumatoid arthritis comprising the step of determining the presence or absence of antibodies directed against C5a-receptor in a sample of the subject to be diagnosed, wherein the presence of antibodies directed against C5a-receptor is indicative of rheumatoid arthritis in said subject. In a further aspect the disclosure relates to a method for diagnosing systemic sclerosis comprising the step of determining the presence or absence of antibodies directed against C5a-receptor in a sample of the subject to be diagnosed, wherein the presence of antibodies directed against C5a-receptor is indicative of systemic sclerosis in said subject. In yet a further aspect the disclosure relates to a method for diagnosing Sjögren's syndrome comprising the step of determining the presence or absence of antibodies directed against C5a-receptor in a sample of the subject to be diagnosed, wherein the presence of antibodies directed against C5a-receptor is indicative of Sjogren's syndrome in said subject.

Furthermore, the disclosure relates to a method for differential diagnosis between systemic lupus erythematodes (SLE) and other autoimmune diseases, e.g. other collagenosis, comprising the steps of (i) determining the level of antibodies directed against C5a-receptor in a sample from a subject to be diagnosed, (ii) comparing the determined level in the sample to either one, two or three of a first, second, and third C5a-receptor antibody control level, a) wherein the first C5a-receptor antibody control level is derived from one or more subjects suffering from an autoimmune disease, e.g. collagenosis, other than SLE, preferably a collagenosis selected from the group consisting of rheumatoid arthritis, systemic sclerosis, and Sjögren's syndrome, and b) wherein the second C5a-receptor antibody control level is derived from one or more subjects suffering from SLE; c) wherein the third C5a-receptor antibody control level is derived from one or more subjects not suffering from an autoimmune disease, preferably not suffering from a collagenosis;
wherein a increased level in the sample from the subject to be diagnosed as compared to the first and third C5a-receptor antibody control level, and/or an equal level as compared to the second C5a-receptor antibody control level is indicative of SLE in said subject to be diagnosed; and
wherein an decreased level in the sample from the subject to be diagnosed as compared to the first and second C5a-receptor antibody control level C5a-receptor, and/or an equal level as compared to the third C5a-receptor antibody control level is indicative of no autoimmune disease, e.g. collagenosis, in said subject to be diagnosed; and
wherein a decreased level as compared to the second C5a-receptor antibody control level, and an increased level as compared to the third C5a-receptor antibody control level, and/or an equal level as compared to the first C5a-receptor antibody control level is indicative of an autoimmune disease, e.g. a collagenosis, other than SLE in said subject to be diagnosed. Preferably a decreased level as compared to the second C5a-receptor antibody control level, and an increased level as compared to the third C5a-receptor antibody control level, and/or an equal level as compared to the first C5a-receptor antibody control level is indicative for an autoimmune disease selected from the group consisting of rheumatoid arthritis, systemic sclerosis, and Sjögren's syndrome.

As outlined herein, it may be desirable to determine levels of antibodies directed against C5a-receptor and compare the levels to control levels. Hence, in a further aspect the present disclosure relates to a method for diagnosing an autoimmune disease, e.g. collagenosis, comprising the steps of (i) determining the level of antibodies directed against C5a-receptor in a sample from a subject to be diagnosed, (ii) comparing the determined level in the sample to a control level of C5a-receptor antibodies derived from one or more subjects without an autoimmune disease, e.g. collagenosis; wherein an increased level in the sample from the subject to be diagnosed as compared to the control level is indicative of an autoimmune disease, e.g. collagenosis, in the subject to be diagnosed. Preferably said collagenosis is selected from the group consisting of systemic lupus erythematosus (SLE), rheumatoid arthritis, and systemic sclerosis, and Sjögren's syndrome, and said control level is derived from one or more subjects without any of said collagenosis.

The skilled person is aware that he also may have to consider further parameters to diagnose the subject. As described herein, the subject to be diagnosed is a mammal, such as a human, dog, cat, sheep, horse, camel, rat, mouse, hamster or guinea pig. The subject is preferably suspected to have an autoimmune disease according to the present disclosure. Preferably the subject to be diagnosed is a human. The subject is preferably a human suspected to have an autoimmune disease, preferably suspected to have a collagenosis; in a further embodiment said subject is suspected to have a collagenosis selected from the group consisting of systemic lupus erythematosus (SLE), rheumatoid arthritis, and systemic sclerosis, and Sjögren's syndrome. The diagnosis to be performed may be dependent on said further parameters. For example a certain patient may have symptoms usually associated with a certain autoimmune disease such as collagenosis. However, as symptoms often may be associated with different diseases, specificity may not be high enough. Hence, the skilled person will instantly appreciate the provision of the method according to the present disclosure which allows to diagnose autoimmune diseases and furthermore to specify symptom-based diagnosis. This may in principle be performed on any autoimmune disease. However, the disease is preferably a collagenosis as defined herein, e.g. selected form the group consisting of systemic lupus erythematosus (SLE), rheumatoid arthritis, and systemic sclerosis, and Sjögren's syndrome. As described herein, the methods are methods for substituting a diagnosis of an autoimmune disease.

In one aspect the present disclosure relates to a method for diagnosing systemic lupus erythematosus (SLE) comprising the steps of (i) determining the level of antibodies directed against C5a-receptor in a sample from a subject to be diagnosed, (ii) comparing the determined level in the sample to a control level of C5a-receptor antibodies derived from one or more subjects without systemic lupus erythematosus (SLE); wherein an increased level in the sample from the subject to be diagnosed as compared to the control level is indicative of systemic lupus erythematosus (SLE) in the subject to be diagnosed. As described herein, the subject to be diagnosed is preferably a subject suspected to have systemic lupus erythematosus (SLE), preferably a human subject suspected to have systemic lupus erythematosus (SLE). The control level in this embodiment is preferably 9 units/ml, more preferably 12 units/ml, even more preferred 15 units/ml. As outlined herein, units may be determined by the skilled person.

In a further aspect the present disclosure relates to a method for diagnosing rheumatoid arthritis comprising the steps of (i) determining the level of antibodies directed against C5a-receptor in a sample from a subject to be diagnosed, (ii) comparing the determined level in the sample to a control level of C5a-receptor antibodies derived from one or more subjects without an autoimmune disease, e.g. collagenosis; wherein an increased level in the sample from the subject to be diagnosed as compared to the control level is indicative of rheumatoid arthritis in the subject to be diagnosed. As described herein, the subject to be diagnosed is preferably a subject suspected to have rheumatoid arthritis, preferably a human subject, suspected to have rheumatoid arthritis. The control level in this embodiment is preferably 9 units/ml, more preferably 10 units/ml, even more preferred 12 units/ml.

In yet a further aspect the present disclosure relates to a method for diagnosing systemic sclerosis comprising the steps of (i) determining the level of antibodies directed against C5a-receptor in a sample from a subject to be diagnosed, (ii) comparing the determined level in the sample to a control level of C5a-receptor antibodies derived from one or more subjects without an autoimmune disease, e.g. collagenosis; wherein an increased level in the sample from the subject to be diagnosed as compared to the control level is indicative of systemic sclerosis in the subject to be diagnosed. As described herein, the subject to be diagnosed is preferably a subject suspected to have systemic sclerosis, preferably a human subject, suspected to have systemic sclerosis. The control level in this embodiment is preferably 9 units/ml, more preferably 10 units/ml, even more preferred 12 units/ml.

In yet a further aspect the present disclosure relates to a method for diagnosing Sjögren's syndrome comprising the steps of (i) determining the level of antibodies directed against C5a-receptor in a sample from a subject to be diagnosed, (ii) comparing the determined level in the sample to a control level of C5a-receptor antibodies derived from one or more subjects without an autoimmune disease, e.g. collagenosis; wherein an increased level in the sample from the subject to be diagnosed as compared to the control level is indicative of Sjögren's syndrome in the subject to be diagnosed. As described herein, the subject to be diagnosed is preferably a subject suspected to have Sjögren's syndrome, preferably a human subject, suspected to have Sjögren's syndrome. The control level in this embodiment is preferably 9 units/ml, more preferably 10 units/ml, even more preferred 12 units/ml.

The antibodies to be detected or determined according to the present disclosure are directed against C5a-receptor. This means that the antibodies specifically bind C5a-receptor. Specific binding of an antibody normally occurs via binding of a binding site of the antigen. The antibodies of the present disclosure are those specifically binding to C5a-receptor or immunogenic fragments thereof. This binding may occur via recognition of sequence or structural epitopes. The skilled person is aware of methods of how to determine specific epitopes, e.g. fragments of the antigen C5a-receptor, which are recognized and specifically bound by the antibodies to be determined. Fragments of C5a-receptor binding to the auto antibodies are called immunogenic or antigenic fragments. The terms "immunogenic" and "antigenic" are used interchangeably herein. Methods for determining fragments of an antigen binding the antibody are described in several publications (e.g. Gershoni, JM; Roitburd-Berman, A; Siman-Tov, DD; Tarnovitski Freund, N; Weiss, Y (2007). "Epitope mapping: The first step in developing epitope-based vaccines". BioDrugs 21 (3): 145-56; Westwood, MR; Hay, FC (2001). Epitope Mapping: a practical approach. Oxford, Oxfordshire: Oxford University Press. ISBN 0-19-963652-4; Flanagan et al. (2011), "Mapping Epitopes with H/D-Ex Mass Spec". Genetic Engineering and Biotechnology news; 31(1); Gaseitsiwe, S.; Valentini, D.; Mahdavifar, S.; Reilly, M.; Ehrnst, A.; Maeurer, M. (2009) "Peptide Microarray-Based Identification of Mycobacterium tuberculosis Epitope Binding to HLA-DRB1*0101, DRB1*1501, and DRB1*0401". Clinical and Vaccine Immunology 17 (1): 168-75; Linnebacher, Michael; Lorenz, Peter; Koy, Cornelia; Jahnke, Annika; Born, Nadine; Steinbeck, Felix; Wollbold, Johannes; Latzkow, Tobias et al. (2012). "Clonality characterization of natural epitope-specific antibodies against the tumor-related antigen topoisomerase IIa by peptide chip and proteome analysis: A pilot study with colorectal carcinoma patient samples" Analytical and Bioanalytical Chemistry 403 (1): 227-38; Cragg, M. S. (2011). "CD20 antibodies: Doing the time warp". Blood 118 (2): 219-20; Banik, Soma S. R.; Doranz, Benjamin J. (2010). "Mapping Complex Antibody Epitopes". Genetic Engineering and Biotechnology News 3 (2): 25-8; and Paes, Cheryl; Ingalls, Jada; Kampani, Karan; Sulli, Chidananda; Kakkar, Esha; Murray, Meredith; Kotelnikov, Valery; Greene, Tiffani A. et al. (2009). "Atomic-Level Mapping of Antibody Epitopes on a GPCR". Journal of the American Chemical Society 131 (20): 6952-4). As described herein, C5a-receptor antibodies are understood as any immunoglobulin specifically recognizing/binding to C5a-receptor. The antibody to be detected in a preferred aspect binds any C5a-receptor, preferably a sequence comprising or consisting of SEQ ID NO: 1.

In the context of the present disclosure the C5a-receptor antibody may particularly be selected from the group of IgA-antibody, IgG-antibody and IgM-antibody, preferably an IgG-antibody, e.g. IgG1, IgG2, IgG3 and IgG4.

The control levels as disclosed herein refer to control levels of antibodies directed against C5a-receptor or an antigenic fragment thereof. It will be readily understood by the skilled person that the control levels from subjects having the desired disease or response as defined in the methods and to which the determined levels are compared to, are not necessarily determined in parallel but may be represented by previously determined levels. Nevertheless, control levels may be determined in parallel. The skilled person with the disclosure as described herein and his knowledge is able to determine such levels, as outlined herein. Hence, the control levels as described herein may be previously defined thresholds. Preferred thresholds are disclosed herein but may also be determined by the person of ordinary skills in the art when considering the disclosure of the present application. Furthermore, it will be acknowledged by the skilled person that control levels are, like the levels to be determined in the subject to be diagnosed, determined in samples of the recited subjects having the desired disease or being healthy, i.e. not having the recited disease. Preferably, the sample is the same kind of sample as the sample of the person to be diagnosed, e.g. when the sample of the latter is serum, the control levels are preferably determined in serum samples derived from the control subjects.

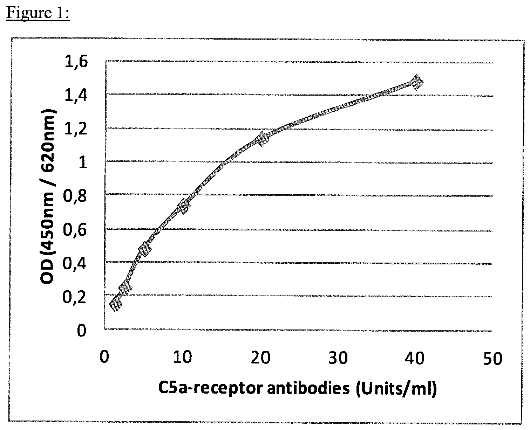

As outlined herein, the levels of antibodies directed against C5a-recptor in a sample of the patient to be diagnosed may be compared to the control groups as defined herein. However, in one aspect the levels are compared to fixed values, i.e. thresholds under or over which a certain diagnosis, or prognosis is given. To this end, unit-standards may be applied. The present inventors set out such standard for the C5a antibodies using one armed serum samples from systemic sclerosis patients. The inventors took a serum sample of a systemic sclerosis patient. However, it will be acknowledged by the skilled person that also other samples may be taken to set a different standard, e.g. samples of patients having other autoimmune diseases, as long as antibodies directed against C5a-receptor are present in an amount sufficient to allow preparation of a standard curve. Nevertheless the principle of generating a standard (units) is the same in any case and is exemplified herein using serum samples of systemic sclerosis patients. As described herein, "units/ml", unless specified otherwise, refers to the concentration of antibodies standardised as exemplified herein. Hence, as described herein, 40 units/ml refers to a dilution of 1:100 of a serum sample of systemic sclerosis patients. The serum sample may be derived from a single patient or of a cohort of a plurality of patients, e.g. a cohort of 200 patients suffering from systemic sclerosis. In one preferred embodiment the standard for the concentrations of the autoimmune antibodies is generated in the following way: a serum sample of a systemic sclerosis patient (or a larger cohort) is diluted (a) 1:100 for standard point 40 Units/ml, (b) 1:200 for standard point 20 Units/ml, (c) 1:400 for standard point 10 Units/ml, (d) 1:800 for standard point 5 Units/ml, (e) 1:1,600 for standard point 2.5 Units/ml and (f) 1:3,200 for standard point 1.25 Units/ml. These standards are then used for the immunoassay chosen, e.g. ELISA, and then correlated with the respective read-out value, e.g. for ELISA the ratio of optical density at 450nm and optical density at 620 nm. A typical standard curve of C5a-receptor auto-antibody ELISA is shown in Figure 1. Nevertheless, the skilled person will readily understand that it may also be possible to standardize the levels of C5a-receptor antibodies using different samples, e.g. samples of patients having other autoimmune diseases.

"Equal" level as described herein means that the levels differ by not more than ± 10 %, preferably by not more than ± 5 %, more preferably by not more than ± 2 %."Decreased" or "increased" level as described herein mean that the levels differ by more than 10 %, preferably by more than 15 %, preferably more than 20 %.

The sample is a sample of a bodily fluid of the subject to be diagnosed. Preferred test samples include blood, serum, plasma, cerebrospinal fluid, urine, saliva, sputum, and pleural effusions. In addition, one of skill in the art would realize that some test samples would be more readily analyzed following a fractionation or purification procedure, for example, separation of whole blood into serum or plasma components. Thus, in a preferred aspect of the disclosure the sample is selected from the group comprising a blood sample, a serum sample, a plasma sample, a cerebrospinal fluid sample, a saliva sample and a urine sample or an extract of any of the aforementioned samples. Preferably, the sample is a blood sample, more preferably a serum sample or a plasma sample. Serum samples particularly preferred samples in the context of the present disclosure.

Where appropriate, the sample may need to be homogenized, or extracted with a solvent prior to use in the present disclosure in order to obtain a liquid sample. A liquid sample hereby may be a solution or suspension. Liquid samples may be subjected to one or more pre-treatments prior to use in the present disclosure. Such pre-treatments include, but are not limited to dilution, filtration, centrifugation, concentration, sedimentation, precipitation, and dialysis. Pre-treatments may also include the addition of chemical or biochemical substances to the solution, such as acids, bases, buffers, salts, solvents, reactive dyes, detergents, emulsifiers, chelators.

"Plasma" as described herein is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (*e.g.* citrated, EDTA or heparinized blood) for at least 15 minutes at 2000 to 3000 g.

"Serum" is the liquid fraction of whole blood that is collected after the blood is allowed to clot. When coagulated blood (clotted blood) is centrifuged serum can be obtained as supernatant. It does not contain fibrinogen, although some clotting factors remain.

In the method of the present disclosure, the antibodies directed against C5a-recpetor are preferably detected in an immunoassay. Suitable immunoassays may be selected from the group of immunoprecipitation, enzyme immunoassay (EIA), enzyme-linked immunosorbenassys (ELISA), radioimmunoassay (RIA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay. Preferably herein the immunoassay is an enzyme linked immunosorbent assay (ELISA).

In the context of the immunoassays as described herein the "C5a-receptor" may be present in its natural cellular environment and can be used together with the material associated with the receptor in its natural state as well as in isolated form with respect to its primary, secondary and tertiary structures. The receptor is well known to those skilled in the art. The protein or its immunogenic (antigenic) fragment is preferably used in isolated form, i.e. essentially free of other proteins, lipids, carbohydrates or other substances naturally associated with the C5a-receptor. "Essentially free of' means that the protein or its immunogenic fragment is at least 75%, preferably at least 85%, more preferably at least 95% and especially preferably at least 99% free of other proteins, lipids, carbohydrates or other substances naturally associated with the C5a-receptor.

As described herein, the naturally occurring protein as well as all modifications, mutants or derivatives of the C5a-receptor can be used. This includes all naturally present modifications as known to the skilled person. Similarly, a C5a-receptor produced by means of recombinant techniques, which includes amino acid modifications, such as inversions, deletions, insertions, additions etc. can be used according to the disclosure provided that this part of the essential function of the C5a-receptor is present, namely the capability of binding antibodies. Such recombinant techniques include the expression of the C5a-receptor in a host cell using an expression vector suited for the selected host cell. The skilled person is able to choose suited host cells and expression vector systems based on his common general knowledge. As the C5a-receptor protein is a trans-membrane protein, membrane extracts of the host cells expressing the receptor can be produced and used as the antigen. One preferred host cell system are Chinese Hamster Ovary cells (CHO cells) with the appropriate expression vector. The C5a-receptor being used may also comprise exceptional amino acids and/or modifications of such as alkylation, oxidation, thiol-modification, denaturation, oligomerization and the like. The C5a-receptor can also be synthesized by chemical means. As described herein, the C5a-receptor particularly can be a protein and/or peptide or a fusion protein, which in addition to other proteins, peptides or fragments thereof, includes the C5a-receptor as a whole or in part. Using conventional methods, peptides or polypeptides of the C5a-receptor which have functionally analogs, analogous properties can be determined by those skilled in the art. For example such polypeptides or peptides have 50-60%, 70% or 80%, preferably 90%, more preferably 95%, and most preferably 98% sequence homology to peptides identified as C5a-receptor, and said homology can be determined, e.g. by means of Smith-Waterman homology search algorithm, using the MPFRCH program (Oxford Molecular), for example.

The term "peptide" or "polypeptide" of an C5a-receptor as used herein , comprises also molecules differing from the original sequence by deletion(s), insertion(s), substitution(s) and/or other modifications well known in the prior art and/or comprising a fragment of the original amino acid molecule, the C5a-receptor still exhibiting the properties mentioned above. Such a peptide has preferably at least a length of 50 residues but may also be shorter, e.g. at least 12, 15, 20 or 25 amino acid residues in length. For example one or more of the extracellular loops may be used. Also included are allele variants and modifications. Methods of producing the above changes in the amino acid sequence are well known to those skilled in the art and have been described in the standard textbooks of molecular biology, e.g. Sambrook et al., Molecular Cloning: A Laboratory Manual, 4th edition (2012); Cold Spring Harbor Laboratory Press, ISBN 978-1-936113-42-2*.* Those skilled in the art will also be able to determine whether to protein or a fragment of C5a-receptor, thus, modified still has the properties mentioned above. The amino acid sequence of C5a-receptor is known. Database entries exist in several well known Databases. When refereeing to the amino acid sequence of C5a-receptor any amino acid sequence known is meant, particularly those disclosed in common databases, preferably of human origin. This protein in humans is encoded by the C5AR gene (Entrez GeneID: 728 (NM_001736.3 and NP_001727.1; Maglott et al. (2005), "Entrez Gene: gene-centered information at NCBI", Nucleic Acids Res. Jan 1, 2005; 33 (Database issue): D54-D58)). A preferred sequence of the C5a-receptor is given herein, i.e. SEQ ID NO: 1. The C5a-receptor may be glycosylated, *in vivo.* In the present specification all of the above illustrated modifications of the C5a-receptor will be referred to as "functionally analogous peptides or proteins" in brief.

The immunoassays can be homogeneous or heterogeneous assays, competitive and non-competitive assays. In a particularly preferred embodiment, the assay is in the form of a sandwich assay, which is a non-competitive immunoassay, wherein the antibodies directed against C5a-receptor (i.e. the "analyte") to be detected and/or quantified are allowed to bind to an immobilized C5a-receptor protein (e.g. comprised in a membrane fraction of CHO cells as exemplified herein) or immunogenic peptide fragments thereof and to a secondary antibody. The C5a-receptor or the immunogenic fragment thereof (i.e. a peptide), may e.g., be bound to a solid phase, e.g. a bead, a surface of a well or other container, a chip or a strip, and the secondary antibody is an antibody which is labeled, e.g. with a dye, with a radioisotope, or a reactive or catalytically active moiety such as a peroxidase, e.g. horseradish peroxidase. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb;10(1):4-10. PMID: 16376134. Sandwich immunoassays can for example be designed as one-step assays or as two-step assays.

The detectable label may for example be based on fluorescence or chemiluminescence. The labelling system comprises rare earth cryptates or rare earth chelates in combination with a fluorescence dye or chemiluminescence dye, in particular a dye of the cyanine type. As described herein, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

As described herein, chemiluminescence based assays comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562, including citations on pages 551-562. Preferred chemiluminescent dyes are acridiniumesters.

The "sensitivity" of an assay relates to the proportion of actual positives which are correctly identified as such, i.e. the ability to identify positive results (true positives positive results / number of positives). Hence, the lower the concentrations of the analyte that can be detected with an assay, the more sensitive the immunoassay is. The "specificity" of an assay relates to the proportion of negatives which are correctly identified as such, i.e. the ability to identify negative results (true negatives / negative results). For an antibody the "specificity" is defined as the ability of an individual antigen binding site to react with only one antigenic epitope. The binding behaviour of an antibody can also be characterized in terms of its "affinity" and its "avidity". The "affinity" of an antibody is a measure for the strength of the reaction between a single antigenic epitope and a single antigen binding site. The "avidity" of an antibody is a measure for the overall strength of binding between an antigen with many epitopes and multivalent antibodies.

An "immunogenic peptide" or "antigenic peptide" as used herein is a portion of the C5a-receptor that is recognized (i.e., specifically bound) by the C5a-receptor antibody. Such immunogenic peptides generally comprise at least 5 amino acid residues, more preferably at least 10, and still more preferably at least 20 amino acid residues of the receptor. However, they may also comprise at least 30, 40, 50, 60, 70, or 74 amino acid residues.

For the purposes of the immunoassays and diagnostic methods as described herein C5a-receptor can be produced by expression in cells, preferably eukaryotic cells or in cell free, preferably eukaryotic cell free systems. Hence, in the assays and methods as described herein the C5a-receptor may be present in its natural cellular environment and can be used together with the material associated with the protein in its natural state as well as in isolated form. Suitable expression systems include Chinese hamster ovary (CHO) cells overexpressing the human C5a-receptor. Hence, cell extracts (particularly extracts from CHO cells overexpressing the human C5a-receptor) can be used to detect anti-CSa-receptor antibodies. As C5a-receptor is membrane bound the cell extract is preferably a membrane extract. Based on the weight of the whole protein or its immunogenic fragment in the preparation (e.g. the "extract") to be used according to the disclosure, the isolated protein should account for at least 0.5%, preferably at least 5% more preferably at least 25%, and in a particular preferred embodiment at least 50%. The protein may be used in isolated form, i.e. essentially free of other proteins, lipids, carbohydrates or other substances naturally associated with the receptor. "Essentially free of' means that the protein is at least 75%, preferably at least 85%, more preferably at least 95% and especially preferably at least 99% free of other proteins, lipids, carbohydrates or other substances naturally associated with the protein.

The disclosure also relates to a method for detecting an anti-CSa-receptor antibody in a sample from a subject, comprising the steps of (a) contacting the sample suspected of comprising an antibody directed against C5a-receptor (anti-C5a-receptor antibody) with C5a-receptor or an antigenic peptide fragment thereof under conditions allowing for the formation of a complex between the anti-CSa-receptor antibody with C5a-receptor or the antigenic peptide fragment thereof, (b) detecting the complex.

The C5a-receptor or the antigenic peptide fragment thereof may preferably be immobilized on a surface. The complex may for example be detected using a secondary antibody against the Fc portion of the anti-CSa-receptor antibody. When the anti-CSa-receptor antibody is an IgG-antibody, the secondary antibody may be an anti-IgG-antibody. Hence, in one embodiment the anti-CSa-receptor antibody to be detected is an IgG-antibody and the secondary antibody is an anti-IgG-antibody, particularly preferred the subject is a human and the secondary antibody is an anti-human-IgG-antibody. The skilled person will understand that it is possible to detect total IgG, i.e. the method does not distinguish between the subtypes of IgG-antibodies which is preferred. Hence, in one aspect the secondary antibody is an anti-human-total IgG-antibody. Nevertheless, in some embodiment it may be preferred that the subtypes are differentially detected. Hence, in a particular aspect, the subject is a human and
(i) the anti-CSa-receptor antibody is an IgG1-antibody and the secondary antibody is an anti-human-IgG1-antibody; or
(ii) the anti-CSa-receptor is an IgG2-antibody and the secondary antibody is an anti-human-IgG2-antibody; or
(iii) the anti-CSa-receptor is an IgG3-antibody and the secondary antibody is an anti-human-IgG3-antibody; or
(iv) the anti-CSa-receptor is an IgG4-antibody and the secondary antibody is an anti-human-IgG4-antibody.

The secondary antibody may for example be labeled with a detectable marker, e.g. a peroxidase.

Furthermore, in the methods of the present disclosure further parameters of the subject may be considered as well for diagnosis, differential diagnosis, etc. Such parameters in a multivariate model may include gender, age, histological evaluation, and other biomarkers. A Cox-Proportional-Hazard regression predicts the dependent variable based on one or more independent variables. These predictors can either be measures (as e.g. level of a biomarker) or categorical data. The skilled person is aware of the fact that diagnostic markers only give a certain degree of sensitivity and specificity, as also outlined herein. He knows that different further parameters might be considered in order to increase both. Nevertheless, the present disclosure provides a new and superior marker for diagnosis, prognosis of an autoimmune disease, particularly for a collagenosis as defined herein. In the context of the methods of the disclosure and particularly the immunoassays, the presence of one or more further diagnostic (bio)markers for the diseases is detected in the sample.

The term "biomarker" (biological marker) relates to measurable and quantifiable biological parameters (*e.g*., specific enzyme concentration, specific hormone concentration, specific gene phenotype distribution in a population, presence of biological substances) which serve as indices for health- and physiology-related assessments, such as disease risk, psychiatric disorders, environmental exposure and its effects, disease diagnosis, metabolic processes, substance abuse, cell line development, epidemiologic studies, etc. Furthermore, a biomarker is defined as a characteristic that is objectively measured and evaluated as an indicator of normal biological processes, or pathogenic processes. A biomarker may be measured on a biosample (as a blood, urine, or tissue test), it may be a recording obtained from a person (blood pressure, ECG, or Holter), or it may be an imaging test. Biomarkers can indicate a variety of health or disease characteristics, including the level or type of exposure to an environmental factor, genetic susceptibility, genetic responses to exposures, biomarkers of subclinical or clinical disease. Thus, a simplistic way to think of biomarkers is as indicators of disease trait (risk factor or risk biomarker), disease state (preclinical or clinical), or disease rate (progression). Accordingly, biomarkers can be classified as antecedent biomarkers (identifying the risk of developing an illness), screening biomarkers (screening for subclinical disease), diagnostic biomarkers (recognizing overt disease), staging biomarkers (categorizing disease severity), or prognostic biomarkers (predicting future disease course, including recurrence. Biomarkers may also serve as surrogate end points. The underlying principle is that alterations in the surrogate end point track closely with changes in the outcome of interest. Surrogate end points have the advantage that they may be gathered in a shorter time frame and with less expense than end points such as morbidity and mortality, which require large clinical trials for evaluation. Additional values of surrogate end points include the fact that they are closer to the exposure/intervention of interest and may be easier to relate causally than more distant clinical events. An important disadvantage of surrogate end points is that if clinical outcome of interest is influenced by numerous factors (in addition to the surrogate end point), residual confounding may reduce the validity of the surrogate end point. It has been suggested that the validity of a surrogate end point is greater if it can explain at least 50% of the effect of an exposure or intervention on the outcome of interest. For instance, a biomarker may be a protein, peptide or a nucleic acid molecule.

The disclosure also relates to the use of the C5a-receptor or an antigenic peptide fragment thereof, preferably as set out herein above, for the diagnosis of an autoimmune disease, preferably for the diagnosis of an collagenosis or glomerulonephritis, more preferably for a collagenosis selected from the group consisting of systemic lupus erythematosus (SLE), rheumatoid arthritis, systemic sclerosis, Sjögren's syndrome, mixed connective tissue disease, eosinophilia infectiosa, polymyositis, dermatomyositis, periarteriitis nodosa, Wegener-granulomatose, CREST-syndrome and SHARP-syndrome, more preferred selected from the group consisting of systemic lupus erythematosus (SLE), rheumatoid arthritis, and systemic sclerosis, and Sjögren's syndrome. The C5a-receptor preferably comprises or consists of SEQ ID NO: 1, or comprising or consisting of a sequence having at least 80 % identity thereto, preferably at least 90 % identity, more preferably at least 95 % identity, even more preferably at least 97 % identity, yet more preferred at least 99 % identity thereto.

As will be apparent to the person skilled in the art, the "diagnostic" method may also being a prognostic method. For example the patho-mechanism of the disease may already being set on but no symptoms of the disease are yet present and/or detectable. Nevertheless, in such case the methods for diagnosis may be used to detect
such "onset" of disease as a prognostic method, i.e. whether the subject will or has the risk to suffer from the disease in future. Hence, the term "method for diagnosis" and "method for prognosis" are to be used synonymously and can be exchanged herein were used.

In the context of the present disclosure, the levels of the anti-C5a-receptor antibodies may be analyzed in a number of fashions well known to a person skilled in the art. For example, each assay result obtained may be compared to a "normal" value, or a value indicating a particular disease or outcome. A particular diagnosis/prognosis may depend upon the comparison of each assay result to such a value, which may be referred to as a diagnostic or prognostic "threshold". In certain aspects, assays for one or more diagnostic or prognostic indicators are correlated to a condition or disease by merely the presence or absence of the indicator(s) in the assay. For example, an assay can be designed so that a positive signal only occurs above a particular threshold concentration of interest, and below which concentration the assay provides no signal above background. In a preferred aspect a level above the 75^{th} percentile of a healthy population is attributed to the presence of the antibody in the sample or indicative of the autoimmune disease according to the disclosure, more preferably above the 95^{th} percentile, or even above the 97.5^{th} percentile.

The sensitivity and specificity of a diagnostic or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy individuals not having an autoimmune disease) and "disease" populations. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, below which the test is considered to be abnormal and above which the test is considered to be normal. The area under the ROC curve is a measure of the probability that the perceived measurement will allow correct identification of a condition. ROC curves can be used even when test results don't necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art. See, *e.g.,* Hanley et al. 1982. Radiology 143: 29-36*.* Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In other aspects, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group.

In the case of a hazard ratio, a value of 1 indicates that the relative risk of an endpoint (e.g., disease) is equal in both the "diseased" and "control" groups; a value greater than 1 indicates that the risk is greater in the diseased group; and a value less than 1 indicates that the risk is greater in the control group.

The skilled artisan will understand that associating a diagnostic or prognostic indicator, with a diagnosis or with a prognostic risk of a future clinical outcome is a statistical analysis. For example, a marker level of lower than X may signal that a patient is more likely to suffer from an adverse outcome than patients with a level more than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, *e.g.,* Dowdy and Wearden, Statistics for Research, John Wiley & Sons, New York, 1983*.* Preferred confidence intervals of the disclosure are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Suitable threshold levels for the stratification of subjects into different groups (categories) can be determined for each particular combination of anti-CSa-receptor antibodies, and disease. This can e.g. be done by grouping a reference population of patients according to their level of anti-C5a-receptor-antibodies into certain quantiles, e.g. quartiles, quintiles or even according to suitable percentiles. For each of the quantiles or groups above and below certain percentiles, hazard ratios can be calculated comparing the risk for an adverse outcome, i.e. an "autoimmune disease", e.g. in terms of disease rate, between those patients who have a certain disease and those who have not. In such a scenario, a hazard ratio (HR) above 1 indicates a higher risk for an adverse outcome for the patients. A HR below 1 indicates beneficial outcome. A HR around 1 (e.g. +/- 0.1) indicates no elevated risk for the particular group of patients. By comparison of the HR between certain quantiles of patients with each other and with the HR of the overall population of patients, it is possible to identify those quantiles of patients who have an elevated risk and thereby stratify subjects according to the present disclosure.

In some cases presence of an autoimmune disease will be detected in patients not showing presence of C5a-receptor antibodies (e.g. in the fifth quintile), while in other cases only patients with presence or increased levels of C5a-receptor antibodies not have an autoimmune disease (e.g. in the first quintile). However, with the above explanations, a skilled person is able to identify those groups of patients having an autoimmune disease. In another aspect of the disclosure, the diagnosis is determined by relating the patient's individual level of marker antibody to certain percentiles (e.g. 75^{th}, 95^{th} or 97.5^{th} percentile) of a healthy population.

Kaplan-Meier estimators may be used for the assessment or prediction of the outcome or risk (e.g. diagnosis, relapse, progression or morbidity) of a patient.

The disclosure also pertains to a research and/or diagnostic kit for the diagnosis of an autoimmune disease e.g. a collagenosis, wherein the kit comprises C5a-receptor or an antigenic peptide fragment thereof. The kit may further comprise means for detecting antibodies binding to said C5a-receptor or antigenic peptide fragment thereof (secondary antibody), e.g. an antibody directed to the Fc portion of the anti-CSa-receptor antibodies to be detected, preferably an anti-human-IgG-antibody. The C5a-receptor preferably comprises or consists of SEQ ID NO: 1 or an antigenic fragment thereof, or comprises or consists of a sequence having at least 80 % identity thereto, preferably at least 90 % identity, more preferably at least 95 % identity, even more preferably at least 97 % identity, yet more preferred at least 99 % identity thereto.

Such kits can comprise a carrier, package or container that is compartmentalized to receive one or more containers such as vials, tubes, and the like, each of the container(s) comprising one of the separate elements to be used in the method. The kit will typically comprise the container described above and one or more other containers comprising materials desirable from a commercial and user standpoint, including buffers, diluents, filters, needles, syringes, and package inserts with instructions for use. In addition, a label can be provided on the container to indicate that the composition is used for a specific therapeutic or non-therapeutic application, and can also indicate directions for either *in vivo* or *in vitro* use, such as those described herein. Directions and/or other information can also be included on an insert which is included with the kit. The disclosure therefore relates to a kit for diagnosing an autoimmune disease as outlined above, said kit comprising C5a-receptor or an antigenic peptide thereof, and means to detect antibodies binding to said C5a-receptor or peptide thereof. Preferably the kit is designed for a method of the present disclosure. The kit is designed to detect autoimmune antibodies in samples of subject and hence comprises means to detect such antibodies, particularly antibodies binding to said C5a-receptor or peptide thereof. Such means are outlined herein above, e.g. for immunoassays. The C5a-receptor or antigenic fragments thereof may be immobilized to a surface, e.g. by using common techniques (e.g. see Giral et al.; *infra*)*.* Further, the kit may comprise compounds to stabilize the C5a-receptor or the antigenic fragment thereof, in particular with respect to its epitopial conformation. In a particular preferred aspect the C5a-receptor or antigenic fragment thereof, e.g. a membrane extract as defined herein, is immobilized on a surface and stabilized, preferably by addition of 1 mM calcium chloride to the buffer in which the receptor is provided. Suited suffers are known to the skilled person and include phosphate suffers, like PBS. The surface to which the receptor is immobilized may be of any suited kind, preferably a material not interfering with complex formation between the receptor and the antibody as well between the C5a-receptor antibody and the secondary antibody. Such materials include plastics, e.g. polysterene. The kits are meant for the detection of autoimmune antibodies in samples of subjects, e.g. blood. Hence, the kit comprise means for the preparation of blood, e.g. for gaining plasma or serum thereof. Furthermore, the kit may comprise control composition and/or standards. The control composition preferably comprises C5a-receptor antibodies as positive control. Furthermore, the kit may comprise one or a plurality of standard compositions. A standard composition comprises C5a-receptor antibodies at a defined concentration. As outlined herein, determination of concentration of autoimmune-antibodies may be performed using standard curves. These curves set out which concentration of antibodies in a sample or solution corresponds to what read-out value of the assay used, e.g. optical density or proportion of optical density at different wavelengths (e.g. 450nm/620nm). To this end the kits may comprise one or more standard compositions having a defined concentration of C5a-receptor antibodies, preferably of the kind to be detected in the method. A standard composition of the kits may for instance comprise C5a-receptor antibodies at concentrations selected from the group consisting of 1.25 units/ml, 2.5 units/ml, 5 units/ml, 10 units/ml, 20 units/ml, and 40 units/ml. In one aspects the kit comprises six standard compositions with the recited concentration. In another aspect the kit comprises one standard composition with the highest concentration of the standard curve or an even higher concentration, such as double or four times the highest concentration of the standard curve, e.g. 40 units/ml or 80 units/ml. The other concentrations may be produced at the side of the end user by further dilutions using suited buffers, e.g. in PBS. A dilution buffer may therefore also be comprised in the kits. The disclosure is further illustrated by the following non-limiting Examples and Figures.

### Disclosed Sequences

### EXAMPLES

### Example 1:

Anti-C5a-receptor autoantibodies in serum samples were measured using a sandwich ELISA kit. To this end, microtiter 96-well polystyrene plates were coated with a membrane extract of CHO cell cultures expressing human full length C5a-receptor of the sequence of SEQ ID NO: 1 using common techniques (e.g. see for AT₁-Receptor ELISA Giral M, Foucher Y, Dufay A, Van Huyen JP, Renaudin K, Moreau A, Philippe A, Hegner B, Dechend R, Heidecke H, Brouard S, Cesbron A, Castagnet S, Devys A, Soulillou JP, Dragun D. Pretransplant sensitization against angiotensin II type 1 receptor is a risk factor for acute rejection and graft loss. Am J Transplant. 2013 Oct;13(10):2567-76). Conformational epitopes of the receptor were maintained by addition of 1 mM calcium chloride to every buffer.

In order to obtain a standard curve, plates were incubated with test sera from an anti-CSa-receptor autoantibody positive index patient suffering from systemic sclerosis. The ELISA was validated according to the FDA's "Guidance for industry: Bioanalytical method validation". C5a-receptor-auto-antibodies are not commercially available; a serum sample from a patient with a systemic sclerosis was used for the standard curve. A 1:100 dilution of the serum sample is defined as 40 units/ml anti-C5a-receptor-antibodies. A 1:100 dilution of a healthy donor (not having an autoimmune disease) served as a negative control (range 9-12 Units/ml). To set a standard for the concentrations of the autoimmune antibodies, a standard curve was generated. In detail, a serum sample of systemic sclerosis serum sample was diluted (a) 1:100 for standard point 40 units/ml, (b) 1:200 for standard point 20 units/ml, (c) 1:400 for standard point 10 units/ml, (d) 1:800 for standard point 5 units/ml, (e) 1:1,600 for standard point 2.5 units/ml and (f) 1:3,200 for standard point 1.25 units/ml. Then the ratio of optical density at 450 nm and 620 nm was determined using the kit and method of above. Each standard point was performed in duplicates. Results are shown in Figure 1.

To maintain the conformational epitopes of the protein/fragment, 1 mM calcium chloride was added to every buffer. Duplicates of a 1:100 dilution of all serum samples were incubated at 4°C for 2 hours. After washing steps using, plates were incubated for 60 minutes with a 1:20.000 dilution of horseradish-peroxidase-labeled goat anti-human-IgG (Cat-No.: 109035008, Jackson, USA) used for detection.

### Example 2:

Anti-C5a-receptor antibody levels in serum samples from 194 healthy donors ("HD"), 143 patients having systemic lupus erythematosus ("SLE"), 21 patients having rheumatoid arthritis ("RA"), 15 patients having systemic sclerosis, and 11 patients having Sjögren's syndrome were measured using the kit and method of Example 1. The levels were determined in units/mL in accordance with the determined standard curve.

**Table 1: Statistical evaluation of the determined C5a-receptor antibodies in serum samples of the patients as indicated. The left column indicates which results were compared (HD: healthy; SLE: systemic lupus erythematosus; SKL: systemic sclerosis; SS: Sjögren's syndrome). Mean values and standard error of mean, as well as the number of samples used, are given for samples A and B. P-values were determined using unpaired t-test.**

| **Serum samples (Samples A : Samples B)** | **Mean ± SEM of Samples A** | **Mean ± SEM of Samples B** | **P value** |
|---|---|---|---|
| HD : SLE | 9,193 ± 0,5339, n=194 | 22,96 ± 1,586, n=143 | < 0,0001 |
| HD : RA | 9,193 ± 0,5339, n=194 | 14,54 ± 3,136, n=21 | 0,0057 |
| HD : SKL | 9,193 ± 0,5339, n=194 | 13,58 ± 3,483, n=15 | 0,0419 |
| HD : SS | 9,193 ± 0,5339, n=194 | 15,62 ± 2,370, n=11 | 0,0060 |
| SLE : RA | 22,96 ± 1,586, n=143 | 14,54 ± 3,136, n=21 | 0,0526 |
| SLE : SKL | 22,96 ± 1,586, n=143 | 13,58 ± 3,483, n=15 | 0,0641 |
| SLE : SS | 22,96 ± 1,586, n=143 | 15,62 ± 2,370, n=11 | 0,2051 |
| RA : SKL | 14,54 ± 3,136, n=21 | 13,58 ± 3,483, n=15 | 0,8403 |
| RA : SS | 14,54 ± 3,136, n=21 | 15,62 ± 2,370, n=11 | 0,8195 |
| SKL : SS | 13,58 ± 3,483, n=15 | 15,62 ± 2,370, n=11 | 0,6586 |

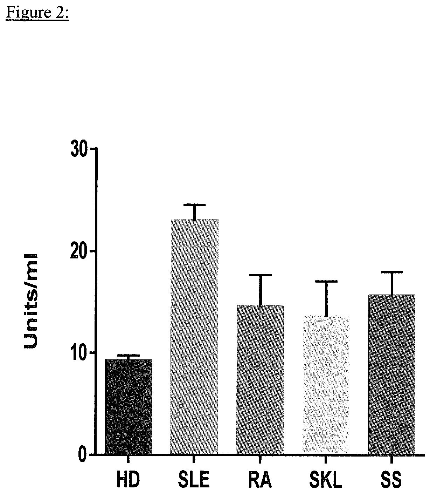

Fig. 2 displays the results given in Table 1. As can be seen, the results are statistically significant.

### Summary

The results of the present Examples show that C5a-receptor antibody levels are significant higher in patients with an autoimmune disease compared to healthy controls. Furthermore, as levels are further increased in samples of patients having systemic lupus erythematosus as compared to other tested autoimmune diseases, a differential diagnosis can be conducted.

## Claims

1. A method for *in vitro* diagnosis of an autoimmune disease, comprising the steps of:
(i) determining the level of autoantibodies directed against Complement component 5a receptor 1 (C5a receptor) in a bodily fluid sample obtained from a subject to be diagnosed, and
(ii) comparing the determined level in the bodily fluid sample to a control level of C5a receptor autoantibodies derived from one or more subjects without an autoimmune disease;
wherein an increased level in the bodily fluid sample from the subject to be diagnosed as compared to the control level is attributed to the presence of autoantibodies directed against C5a-receptor and is indicative of an autoimmune disease in the subject to be diagnosed.

2. The method for *in vitro* diagnosis of an autoimmune disease according to claim 1, wherein a level of C5a-receptor autoantibodies in the bodily fluid sample of the patient to be diagnosed of more than 1.2 fold as compared to the control level is indicative of an autoimmune disease in the subject to be diagnosed.

3. The method for *in vitro* diagnosis of an autoimmune disease according to claim 1, wherein the level of autoantibodies directed against C5a-receptor is determined in a bodily fluid sample from a subject to be diagnosed, and wherein a level of autoantibodies directed against C5a-receptor above 9 units/ml is indicative of an autoimmune disease in said subject.

4. The method according to any one of claims 1 to 3, wherein the autoimmune disease is a collagenosis or glomerulonephritis.

5. The method for diagnosing an autoimmune disease according to claim 4, wherein said collagenosis is selected from the group consisting of systemic lupus erythematosus (SLE), rheumatoid arthritis, scleroderma, Sjögren's syndrome, mixed connective tissue disease, systemic sclerosis, eosinophilia infectiosa, polymyositis, dermatomyositis, periarteriitis nodosa, Wegener-granulomatose, CREST-syndrome, and SHARP-syndrome

6. The method according to any one of claims 1 to 5, wherein said C5a receptor autoantibodies are detected in an immunoassay.

7. The method according to claim 6, wherein the immunoassay is selected from the group consisting of immunoprecipitation, enzyme immunoassay (EIA), radioimmunoassay (RIA), enzyme-linked immunosorbent assay (ELISA), fluorescent immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay or luminex.

8. The method according to any one of the preceding claims, wherein the bodily fluid sample is a plasma or serum sample.

9. The method according to any one of the preceding claims, comprising the steps of
(a) contacting the bodily fluid sample with the C5a-receptor or an antigenic peptide fragment thereof under conditions allowing for the formation of a complex between anti-C5a-receptor autoantibodies and C5a-receptor or said antigenic peptide fragment thereof, and
(b) detecting the complex.

10. The method of claim 9, wherein the C5a-receptor or the antigenic peptide fragment thereof is immobilized on a surface.

11. The method according to any one of claims 9 or 10, wherein the complex is detected using a secondary antibody against the Fc portion of the C5a-receptor autoantibody, wherein the C5a-receptor autoantibody is an IgG antibody and the secondary antibody is an anti-IgG-antibody, and wherein the secondary antibody is labeled with a detectable marker.

12. The method of any or the preceding claims, wherein the presence of one or more further markers for an autoimmune disease is detected in the bodily fluid sample.

13. Use of C5a-receptor or an antigenic peptide fragment thereof for the *in vitro* diagnosis of an autoimmune disease, wherein said C5a-receptor or said antigenic peptide fragment thereof is used for detection of C5a-receptor autoantibodies in a bodily fluid sample of the subject to be diagnosed.

## Patentansprüche

1. Verfahren zur *in vitro*-Diagnose einer Autoimmunerkrankung, umfassend die Schritte:
(i) Bestimmen der Spiegel von Autoantikörpern, die gegen den Rezeptor 1 der Komplementkomponente 5a (C5a-Rezeptor) gerichtet sind, in einer Probe einer Körperflüssigkeit, die von einem zu diagnostizierenden Individuum erhalten wurde, und
(ii) Vergleichen des bestimmten Spiegels in der Körperflüssigkeitsprobe mit einem Kontrollspiegel von C5a-Rezeptor-Autoantikörpern, die von einem oder mehreren Individuen ohne eine Autoimmunerkrankung stammen;
wobei ein im Vergleich zum Kontrollspiegel erhöhter Spiegel in der Körperflüssigkeitsprobe des zu diagnostizierenden Individuums dem Vorliegen von Autoantikörpern, die gegen den C5a-Rezeptor gerichtet sind, zugeschrieben wird und ein Anzeichen für eine Autoimmunerkrankung bei dem zu diagnostizierenden Individuum ist.

2. Verfahren zur *in vitro*-Diagnose einer Autoimmunerkrankung nach Anspruch 1, wobei ein im Vergleich zum Kontrollspiegel mehr als 1,2-facher Spiegel von C5a-Rezeptor-Autoantikörpern in der Körperflüssigkeitsprobe des zu diagnostizierenden Patienten ein Anzeichen für eine Autoimmunerkrankung bei dem zu diagnostizierenden Individuum ist.

3. Verfahren zur *in vitro*-Diagnose einer Autoimmunerkrankung nach Anspruch 1, wobei der Spiegel von Autoantikörpern, die gegen den C5a-Rezeptor gerichtet sind, in einer Körperflüssigkeitsprobe von einem zu diagnostizierenden Individuum bestimmt wird, und wobei ein Spiegel von Autoantikörpern, die gegen den C5a-Rezeptor gerichtet sind, der über 9 Einheiten/ml liegt, ein Anzeichen für eine Autoimmunerkrankung bei dem Individuum ist.

4. Verfahren nach einem der Ansprüche 1 bis 3, wobei die Autoimmunerkrankung eine Kollagenose oder Glomerulonephritis ist.

5. Verfahren zur Diagnose einer Autoimmunerkrankung nach Anspruch 4, wobei die Kollagenose ausgewählt ist aus der Gruppe bestehend aus systemischem Lupus Erythematodes (SLE), rheumatoider Arthritis, Sklerodermie, Sjögren's Syndrom, Mischkollagenose, systemischer Sklerose, Eosinophilia infectiosa, Polymyositis, Dermatomyositis, Periarteriitis nodosa, Wegener-Granulomatose, CREST-Syndrom und SHARP-Syndrom.

6. Verfahren nach einem der Ansprüche 1 bis 5, wobei die C5a-Rezeptor-Autoantikörper in einem Immunassay nachgewiesen werden.

7. Verfahren nach Anspruch 6, wobei der Immunassay ausgewählt ist aus der Gruppe bestehend aus Immunpräzipitation, Enzymimmunassay (EIA), Radioimmunassay (RIA), Enzyme-linked Immunosorbent Assay (ELISA), Fluoreszenzimmunassay, einem Chemilumineszenzassay, einem Agglutination-Assay, nephelometrischem Assay, turbidimetrischem Assay, einem Western Blot, einem kompetitiven Immunassay, einem nichtkompetetiven Immunassay, einem homogenen Immunassay, einem heterogenen Immunassay, einem Bioassay und einem Reporterassay wie einem Luziferase-Assay oder Luminex.

8. Verfahren nach einem der vorangehenden Ansprüche, wobei die Körperflüssigkeitsprobe eine Plasma- oder Serumprobe ist.

9. Verfahren nach einem der vorangehenden Ansprüche, umfassend die Schritte:
(a) Inkontaktbringen der Körperflüssigkeitsprobe mit dem C5a-Rezeptor oder einem antigenischen Peptidfragment davon unter Bedingungen, die die Bildung eines Komplexes aus Anti-C5a-Rezeptor-Autoantikörpern und C5a-Rezeptor oder dem antigenischen Peptidfragment davon ermöglichen, und
(b) Nachweisen des Komplexes.

10. Verfahren nach Anspruch 9, wobei der C5a-Rezeptor oder das antigenische Peptidfragment davon auf einer Oberfläche immobilisiert ist.

11. Verfahren nach einem der Ansprüche 9 oder 10, wobei der Komplex unter Verwendung eines sekundären Antikörpers gegen den Fc-Teil des C5a-Rezeptor-Autoantikörpers nachgewiesen wird, wobei der C5a-Rezeptor-Autoantikörper ein IgG-Antikörper ist und der sekundäre Antikörper ein Anti-IgG-Antikörper ist, und wobei der sekundäre Antikörper mit einem nachweisbaren Marker markiert ist.

12. Verfahren nach einem der vorangehenden Ansprüche, wobei das Vorliegen von einem oder mehrerer weiterer Marker für eine Autoimmunerkrankung in der Körperflüssigkeitsprobe nachgewiesen wird.

13. Verwendung eines C5a-Rezeptors oder eines antigenischen Peptidfragments davon für die *in vitro*-Diagnose einer Autoimmunerkrankung, wobei der C5a-Rezeptor oder das antigenische Peptidfragment davon zum Nachweis von C5a-Rezeptor-Autoantikörpern in einer Körperflüssigkeitsprobe des zu diagnostizierenden Individuums verwendet wird.

## Revendications

1. Procédé pour le diagnostic *in vitro* d'une maladie auto-immune, comprenant les étapes de :
(i) détermination du niveau d'autoanticorps dirigés contre le récepteur 1 du composant du complément 5a (récepteur de C5a) dans un échantillon de liquide biologique obtenu auprès d'un sujet destiné à être diagnostiqué, et
(ii) comparaison du niveau déterminé dans l'échantillon de liquide biologique à un niveau témoin d'autoanticorps pour le récepteur de C5a dérivés d'un ou plusieurs sujets sans maladie auto-immune ;
où un niveau accru dans l'échantillon de liquide biologique provenant du sujet destiné à être diagnostiqué comparé au niveau témoin est attribué à la présence d'autoanticorps dirigés contre le récepteur de C5a et est une indication d'une maladie auto-immune chez le sujet destiné à être diagnostiqué.

2. Procédé pour le diagnostic *in vitro* d'une maladie auto-immune selon la revendication 1, où un niveau d'autoanticorps pour le récepteur de C5a dans l'échantillon de liquide biologique du patient destiné à être diagnostiqué de plus de 1,2 fois comparé au niveau témoin est une indication d'une maladie auto-immune chez le sujet destiné à être diagnostiqué.

3. Procédé pour le diagnostic *in vitro* d'une maladie auto-immune selon la revendication 1, où le niveau d'autoanticorps dirigés contre le récepteur de C5a est déterminé dans un échantillon de liquide biologique provenant d'un sujet destiné à être diagnostiqué, et où un niveau d'autoanticorps dirigés contre le récepteur de C5a supérieur à 9 unités/ml est une indication d'une maladie auto-immune chez ledit sujet.

4. Procédé selon l'une quelconque des revendications 1 à 3, où la maladie auto-immune est une collagénose ou une glomérulonéphrite.

5. Procédé pour diagnostiquer une maladie auto-immune selon la revendication 4, où ladite collagénose est choisie dans le groupe consistant en le lupus érythémateux systémique (LES), la polyarthrite rhumatoïde, la sclérodermie, le syndrome de Sjögren, une maladie du tissu conjonctif mixte, la sclérose systémique, l'éosinophilie infectieuse, la polymyosite, la dermatomyosite, la périartérite noueuse, la granulomatose de Wegener, le syndrome de CREST et le syndrome de SHARP.

6. Procédé selon l'une quelconque des revendications 1 à 5, où lesdits autoanticorps pour le récepteur de C5a sont détectés dans un immunotest.

7. Procédé selon la revendication 6, où l'immunotest est choisi dans le groupe consistant en l'immunoprécipitation, un immunotest enzymatique (EIA), un radioimmunotest (RIA), un test d'immunoabsorption à enzyme liée (ELISA), un immunotest fluorescent, un test chimiluminescent, un test d'agglutination, un test néphélométrique, un test turbidimétrique, un transfert de Western, un immunotest compétitif, un immunotest non compétitif, un immunotest homogène, un immunotest hétérogène, un biotest et un test à rapporteur comme un test à la luciférase ou luminex.

8. Procédé selon l'une quelconque des revendications précédentes, où l'échantillon de liquide biologique est un échantillon de plasma ou de sérum.

9. Procédé selon l'une quelconque des revendications précédentes, comprenant les étapes de
(a) mise en contact de l'échantillon de liquide biologique avec le récepteur de C5a ou un fragment de peptide antigénique de celui-ci dans des conditions permettant la formation d'un complexe entre les autoanticorps anti-récepteur de C5a et le récepteur de C5a ou ledit fragment de peptide antigénique de celui-ci, et
(b) détection du complexe.

10. Procédé selon la revendication 9, où le récepteur de C5a ou le fragment de peptide antigénique de celui-ci est immobilisé sur une surface.

11. Procédé selon l'une quelconque des revendications 9 ou 10, où le complexe est détecté au moyen d'un anticorps secondaire contre la partie Fc de l'autoanticorps pour le récepteur de C5a, où l'autoanticorps pour le récepteur de C5a est un anticorps IgG et l'anticorps secondaire est un anticorps anti-IgG, et où l'anticorps secondaire est marqué avec un marqueur détectable.

12. Procédé selon l'une quelconque des revendications précédentes, où la présence d'un ou plusieurs marqueurs supplémentaires pour une maladie auto-immune est détectée dans l'échantillon de liquide biologique.

13. Utilisation du récepteur de C5a ou d'un fragment de peptide antigénique de celui-ci pour le diagnostic *in vitro* d'une maladie auto-immune, où ledit récepteur de C5a ou ledit fragment de peptide antigénique de celui-ci est utilisé pour la détection d'autoanticorps pour le récepteur de C5a dans un échantillon de liquide biologique du sujet destiné à être diagnostiqué.
